# EUROPEAN PATENT APPLICATION

(11) **EP 3 342 449 A1**
(43) Date of publication of application: **04.07.2018**
(21) Application number: 17150006.9
(22) Date of filing: 02.01.2017
(51) Int. Cl.: A61M 39/02, A61M 39/10, F16K 15/14, A61M 39/24

(54) **PORT DEVICE FOR INJECTING A MEDICAL FLUID INTO A MEDICAL LINE**

(71) Applicant: Fresenius Kabi Deutschland GmbH, 61352 Bad Homburg (DE)
(72) Inventor: PÜTTER, Harry, 36364 Bad Salzschlirf (DE)
(74) Representative: Kusche, Robert

(57) **Abstract**

A port device (1) for injecting a medical fluid into a medical line (2, 3) comprises a body (13), a channel (14) arranged in the body (13), a first connector (10) arranged at a first end (140) of the channel (14) for connecting a first medical line (2) to the port device (1), and a second connector (11) arranged at a second end (141) of the channel (14) for connecting a second medical line (3) to the port device (1). Furthermore, the port device (1) comprises a port (12) for connecting an injection device (4) to the port device (13) for injecting a medical fluid into the channel (14), the port (12) having a conduit (124) being in fluid connection with the channel (14), and a closing element (15) which in a first state separates the conduit (124) of the port (12) from the channel (14) and is elastically deformable from the first state to allow a fluid passage from the conduit (124) into the channel (14). In this way, a port device is provided which is easy to use and effectively hinders a misuse for opening the port device.

## Description

The invention relates to a port device for injecting a medical fluid into a medical line according to the preamble of claim 1.

A port device of this kind comprises a body, a channel arranged in the body, a first connector arranged at a first end of the channel for connecting a first medical line to the port device, and a second connector arranged at a second end of the channel for connecting a second medical line to the port device.

A medical line of this kind may for example be a line used for the enteral feeding of a patient. Enteral feeding generally refers to the delivery of a nutritionally complete feed, containing protein, carbohydrate, fat, water, minerals and vitamins, directly into the stomach, duodenum or jejunum of a patient. A feeding tube, for this purpose, may for example be passed through the nares (nostril), down the esophagus and into the stomach (so-called nasogastric feeding tube). A nasojejunal feeding tube, in comparison, may be passed further through the stomach into the jejunum, the middle section of the small intestine. And a gastric feeding tube is a tube inserted through a small incision in the abdomen into the stomach and is used, preferably, for long-term enteral nutrition.

Within enteral feeding systems, it may be required to inject an additional medical fluid, such as a medication or an additional feeding solution, into a feeding line. There hence is a need for a port device to be arranged in a feeding line for example for the enteral feeding, the port device allowing for an access to the feeding line to inject a medical fluid into the feeding line during a feeding procedure.

Because existing port devices are bulky in their design and may be unintentionally opened for example by a movement of the patient or by a manipulation by a non-approved user, for example a child, there is a desire for a port device which grants an easy access to a feeding line, but at the same time avoids a misuse which may lead to an unintentional opening of a port. The unintentional opening of the port otherwise may allow for an entrance of contaminating substances into the feeding line.

It is an object of the instant invention to provide a port device which is easy to use and effectively hinders a misuse for opening the port device.

The object is achieved by means of a port device according to the features of claim 1.

A port device of this kind comprises a port for connecting an injection device to the port device for injecting a medical fluid into the channel, the port having a conduit being in fluid connection with the channel. The port device furthermore comprises a closing element, which in a first state separates the conduit of the port from the channel and is elastically deformable from the first state to allow a fluid passage from the conduit into the channel.

The port device hence provides a port having the function of a valve. The port comprises a conduit through which a medical fluid, such as a medication or an additional feeding solution, may be injected into the medical line. In a non-used state, the conduit is closed such that contaminating substances may not enter via the conduit into the channel of the port device. In the non-used state a closing element closes the conduit and separates it from the channel such that a fluid transfer from the conduit into the channel is prevented. From the non-used, first state, the closing element can be elastically deformed such that a passage from the conduit into the channel is opened, allowing for a fluid transfer from the conduit into the channel for injecting a medical fluid into the channel and hence into a medical line connected to the channel.

The connectors of the port device may for example be so called ENfit connectors, ENfit providing for a standard connector system for the enteral feeding, allowing to connect components dedicated to the enteral feeding, but preventing a connection of other components not dedicated to the enteral feeding. The first connector and the second connector allowing for connecting medical lines to the port device accordingly may be male or female ENfit connectors. In addition, also a connector at the port for connecting an injection device to the port device may be an ENfit connector.

It is to be noted, however, that in principle any suitable connectors for releasably or non-releasably connecting medical lines to the port device may be used.

The closing element may for example comprise a closing pin which, in the first state of the closing element, reaches into the conduit of the port. Via the closing pin, hence, the closing element closes the conduit against a fluid transfer from the conduit into the channel of the port device. The conduit herein may have the shape of a hollow cylindrical column. The closing pin in this regard may also have a cylindrical shape.

The closing element can be deformed from its first state in order to open the conduit and to allow for a fluid passage from the conduit into the channel. By elastically deforming the closing element, the closing pin can be removed from the conduit such that, in a second state, it no longer closes the conduit, hence allowing for a fluid passage from the conduit into the channel.

The closing pin may be for example arranged on a body section of the closing element. The body section herein, in one embodiment, is elastically connected to the body of the port device for example by means of a membrane section extending from the body section towards the body of the port device. When transferring the closing element from its first state (in which it closes the conduit against a fluid passage from the conduit into the channel) towards the second state (in which a fluid passage from the conduit into the channel is opened), the membrane section is elastically tensioned, for example by an injection pressure caused by a fluid injected into the port or mechanically by acting onto the closing element for example using an injection device in the shape of a syringe. If the injection of the medical fluid is concluded and the injection device is removed from the port, the closing element, due to the elastic tensioning of the membrane section, automatically resets into its non-deformed, first state, in which the conduit again is closed towards the channel.

The membrane section may, in one embodiment, extend from the body section of the closing element in order to elastically connect the body section with the body of the port device. Herein, the membrane section may comprise one or multiple openings through which a fluid may flow, such that a fluid passage from the conduit into the channel is possible when the closing element is in its deformed, second state.

The closing element, in one embodiment, is placed inside the channel and hence closes the conduit at an end leading into the channel. For this, the membrane section may for example be connected, along a circumferential fastening edge, to the inside wall of the body of the port device. The closing element herein can be deformed by displacing it in an injection direction, hence removing the closing element from the opening end of the conduit and hence allowing for a fluid transfer from the conduit into the channel.

The port may have the shape of a connector, in particular an ENfit connector, allowing for a connection of an injection device to the port device. Herein, the port for example may comprise a pin having a substantially cylindrical shape, the conduit being formed axially in the pin. The pin may for example be part of a female connector and may be engageable with a complementary connector of the injection device. On the pin, for example, a threading may be formed allowing to screw on the connector of the injection device such that a locking connection between the injection device and the port of the port device is established.

The body of the port device may have a substantially longitudinal shape, the channel being formed within the body of the port device extending along the longitudinal axis through the body. The conduit herein may be formed in the body extending along a substantially transverse direction with respect to the channel. The connectors allowing to connect medical lines to the port device are arranged at far ends of the body (the opposing ends along the longitudinal axis of the body), whereas the port is arranged at a middle section of the body.

The port device, with its body, may for example be formed from plastics using an injection molding technique. The body herein has a substantially rigid constitution. The closing element, in contrast, is elastically deformable and may be formed as a membrane element from a soft elastic material, such as an elastomer.

The idea underlying the invention shall subsequently be described in more detail with respect to the embodiments shown in the figures. Herein,
- Fig. 1: shows a schematic drawing of a patient, with an enteral feeding line connected to the patient for the enteral feeding;
- Fig. 2: shows a perspective view of a port device to be used on an enteral feeding line;
- Fig. 3: shows a sectional view of the port device;
- Fig. 4: shows a partial view of the port device, with a closing element being opened to allow for a fluid passage from a conduit of a port into a channel of the port device;
- Fig. 5: shows a schematic view of the closing element within the port device:
- Fig. 6: shows another embodiment of a closing element; and
- Fig. 7: shows a schematic view of a different embodiment of a port device.

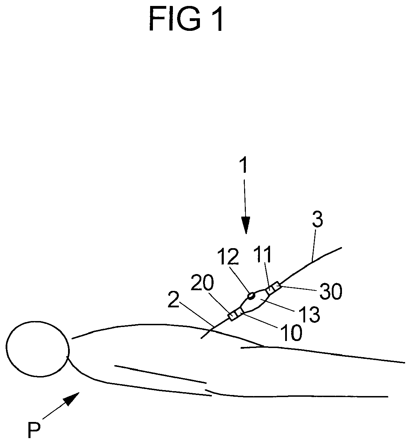

Fig. 1 shows a schematic drawing of a patient P subjected to an enteral feeding procedure. A patient line 2 is for example via a small incision in the abdomen inserted into the stomach of the patient P for delivering a nutritionally complete feed, containing protein, carbohydrate, fat, water, minerals and vitamins, directly into the stomach, duodenum or jejunum of the patient P. The patient line 2 is in connection with a feeding line 3, the feeding line 3 for example being connected to a delivery device such as a pumping device via which the feeding solution is delivered for example from a container towards the patient P.

In between the feeding line 3 and the patient line 2, a port device 1 is placed, the port device 1 having a body 13 and a port 12 arranged thereon, allowing for an injection of a medical fluid into the feeding line system, in order to add the medical fluid to the feeding solution. The medical fluid may for example comprise a medication or an additional feeding solution, hence adding a substance to the feeding solution fed to the patient P via the lines 2, 3.

The port device 1 is connected, via connectors 10, 11 arranged on the body 13, to connectors 20, 30 of the lines 2, 3 and hence establishes a fluid connection from the line 3 into the line 2. A feeding solution may pass from the line 3 through the body 13 of the port device 1 into the line 2, the port 12 arranged on the body 13 allowing for an access to add an additional medical fluid to the feeding solution fed from the line 3 towards the line 2.

A first embodiment of a port device 1 is shown in Fig. 2.

The port device 1 comprises a body 13 having a substantially longitudinal shape, connectors 10, 11 being arranged at opposing ends of the body 13.

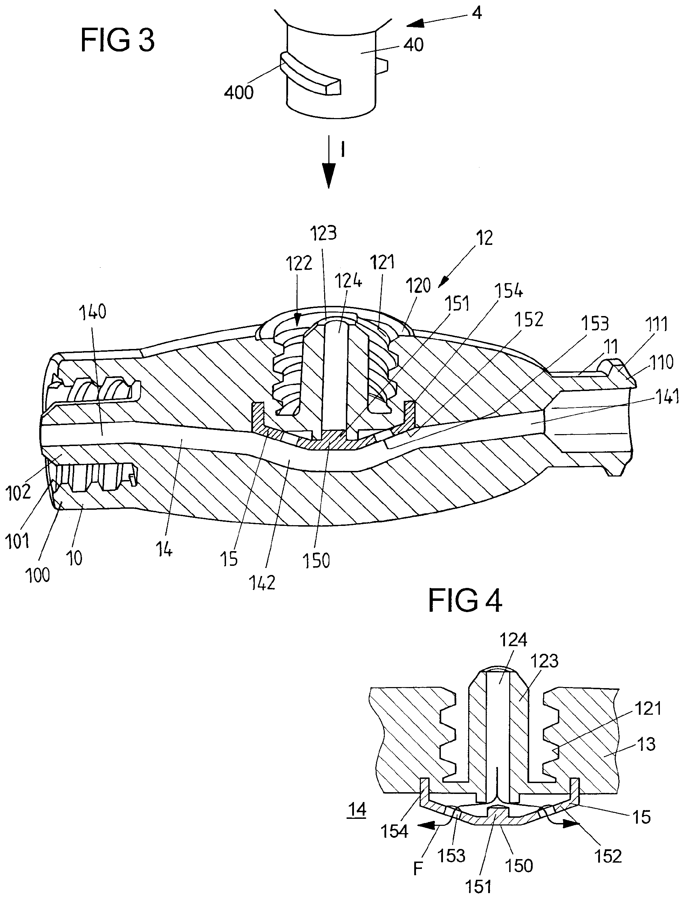

A first connector 10 herein has the shape of a female ENfit connector, having an outer shaft 100 and an inner pin 102 defining a radial space in between. At the inner circumferential wall of the shaft 100 a threading 101 is formed, allowing for screwing on a connector of an associated line 2 in order to establish a fluid connection between the line 2 and a channel 14 formed within the body 13, as illustrated in Fig. 3.

The other, second connector 11 has the shape of a male ENfit connector and comprises a shaft 110 with a threading 111 arranged on a circumferential outer wall of the shaft 110. The connector 11 may be connected with a female ENfit connector of an associated medical line 3, allowing for a screwing connection of the port device 1 to the line 3 in order to establish a fluid connection between the channel 14 in the body 13 and the line 3.

As visible in the sectional view of Fig. 3, the channel 14 longitudinally extends through the body 13, a first end 140 of the channel 14 reaching into the pin 102 of the connector 10 and a second end 141 opening into the shaft 110 of the connector 11. The channel 14 widens in a central region 142, the central region 142 presenting an entrance region associated with the port 12 and allowing for an entrance of an injected medical fluid through a conduit 124 of the port 12.

The port 12, in the embodiment of Fig. 2 and 3, has the shape of a female ENfit connector and comprises an outer wall 120, at the inside of which a threading 121 is arranged. Coaxially to the outer wall 120 and spaced at a radial distance to the outer wall 120 a pin 123 is arranged, the pin 123 forming a radial space 122 with the outer wall 120. The conduit 124 extends through the pin 123 and opens into the channel 14, hence allowing for injecting a medical fluid into the channel 14.

To the port 12 a connector 40 in the shape of a male ENfit connector of an injection device 4 may be connected. The injection device 4 may for example have the shape of a syringe, hence allowing for injecting a medical fluid contained for example in a cylindrical tube of the syringe into the channel 14 and hence into the feeding line system provided by the lines 2, 3.

The connector 40 comprises a threading 400, such that the connector 14 can be inserted in an insertion direction I into the space 122 of the port 12 and can be screwed about the insertion direction I into the port 12 in order to establish a screw-type, locking connection between the connector 40 and the port 12.

The conduit 124 extends substantially transverse to the channel 14 and opens into the central region 142 of the channel 14. In a non-used state of the port device 1, the conduit 124 herein is closed relative to the channel 14 by means of a closing element 15 in the shape of a membrane element, the closing element 15 comprising a body section 150 with a closing pin 151 arranged thereon. The closing pin 151, in the non-used state of the port device 1, reaches into the end of the conduit 124 facing the channel 14, such that the conduit 124 is effectively closed with respect to the channel 14, hence preventing the entrance of substances from the conduit 124 into the channel 14.

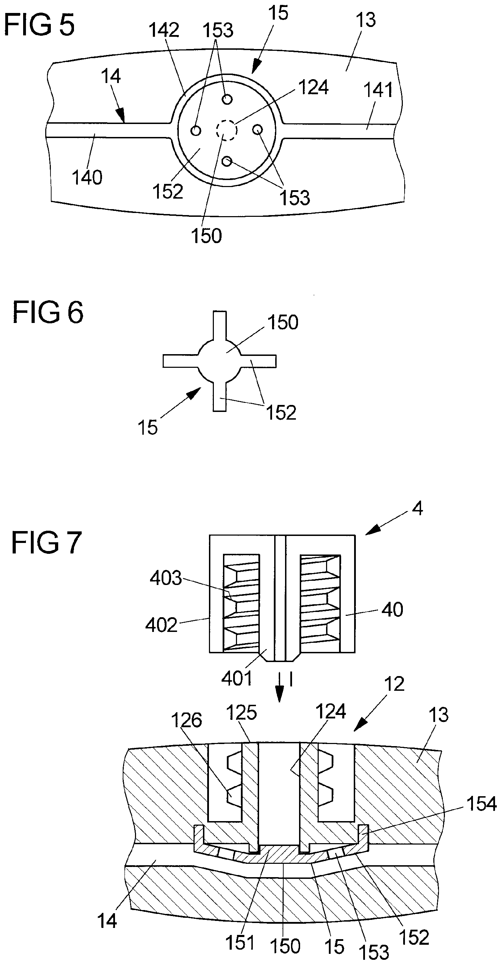

The body section 150 of the closing element 15 is connected, inside the central region 142 of the channel 14, to an inside wall of the body 13 via a membrane section 152, the membrane section 152 extending from the body section 150 and being fastened to the body 13 along a circumferential fastening edge 154. As schematically illustrated in Fig. 5, the closing element 15 with its membrane section 152 extending from the body section 150 (in a top view) may have a substantially circular shape, the membrane section 152 circumferentially enclosing the body section 150 and circumferentially connecting the body section 150 to the inside wall of the body 13.

The closing element 15 is elastically deformable from its closing, first state, illustrated in Fig. 3. The elastic deformation of the closing element 15, as illustrated in Fig. 4, herein is caused by an injection pressure of a medical fluid injected via the port 12 into the conduit 124 by an injection device 4 connected to the port 12. If the pressure in the conduit 124 is sufficiently large, the closing element 15 will, under elastic tensioning of the membrane section 152, be deformed such that the closing pin 151 is removed from the conduit 124, hence opening a fluid passage from the conduit 124 into the channel 14. The membrane section 152 comprises a multiplicity of openings 153, as illustrated in Fig. 4 and 5, such that a fluid flow F may pass from the conduit 124 into the channel 14 and towards the line 2.

If the injection device 4 is again removed, the closing element 15, due to the tensioning of the membrane section 152, will automatically revert to its non-deformed, first state in which it closes again the conduit 124 with respect to the channel 14, such that no substances may enter via the conduit 124 into the channel 14. The port 12 hence again is closed, such that the entrance of contaminating substances into the channel 14 is prevented.

The port 12 hence substantially has the function of a valve, which easily can be opened by connecting a suitable injection device 4 to the port 12, allowing for an injection of medical fluids into the channel 14 and hence into the feeding line system of the lines 2, 3. If no injection device 4 is connected to the port 12, the conduit 124 is automatically closed, hence preventing a contamination of an enteral feed provided via the lines 2, 3.

In the embodiment of Fig. 3 to 5, the closing element 15 has a planar membrane section 152 comprising openings 153. In an alternative embodiment, illustrated in Fig. 6, the body section 150 of the closing element 15 may be connected to the body 13 via elastically deformable webs providing connection sections 152.

In an alternative embodiment of a port device 1, schematically illustrated in Fig. 7, the port 12 has the shape of a male ENfit connector, allowing for connecting a connector 40 in the shape of a female ENfit connector of an injection device 4. The port 12 comprises a shaft 125, the conduit 124 being formed therein. At an outer wall of the shaft 125, a threading 126 is arranged. The connector 40 of the injection device 4, in turn, comprises an outer shaft 402 having a threading 403 at an inner circumferential wall. A pin 401 is coaxially placed with respect to the outer shaft 402 and forms a radial space together with the outer shaft 402, in which the shaft 125 of the port 12 may be received.

The connector 14, in the embodiment of Fig. 7, can be inserted in the insertion direction I into the port 12 and can be screwed onto the shaft 125 of the port 12 about the insertion direction I. When inserting the connector 14 into the port 12, the pin 401 of the connector 40 enters into the conduit 124 and, with its tip, acts onto the closing pin 151 of the closing element 15, hence mechanically removing the closing pin 151 from the conduit 124 and opening the conduit 124 for a fluid passage into the channel 14. A medical fluid may hence be injected from the injection device 4 via the conduit 124 into the channel 14.

Other than the different design of the port 12, the port device 1 of the embodiment of Fig. 7 is functionally equivalent to the previously described embodiment, such that it shall be referred to the above.

The idea underlying the invention is not limited to the embodiments described above, but may be implemented in an entirely different fashion.

In particular, a port device as described herein, in principle, is not limited for a use for the enteral feeding, but may be used also on other medical lines, for example for the parenteral feeding, the transfusion or the infusion.

Also, the specific connectors described herein are not limiting for the invention. In principle, the port device may comprise any suitable connectors for connecting any suitable medical line and/or injection device to the port device.

### List of Reference Numerals

- 1: Port device
- 10: Connector
- 100: Shaft
- 101: Threading
- 102: Pin
- 11: Connector
- 110: Shaft
- 111: Threading
- 12: Port
- 120: Wall
- 121: Threading
- 122: Space
- 123: Pin
- 124: Conduit
- 125: Shaft
- 126: Threading
- 13: Body
- 14: Channel
- 140, 141: End
- 142: Entrance region
- 15: Closing element
- 150: Body section
- 151: Closing pin
- 152: Membrane section
- 153: Opening
- 154: Fastening section
- 2, 3: Line
- 20, 30: Connector
- 4: Injection device (syringe)
- 40: Connector
- 400: Threading
- 401: Pin
- 402: Shaft
- 403: Threading
- F: Flow
- I: Insertion direction
- P: Patient

## Claims

1. Port device (1) for injecting a medical fluid into a medical line (2, 3), comprising:
- a body (13),
- a channel (14) arranged in the body (13),
- a first connector (10) arranged at a first end (140) of the channel (14) for connecting a first medical line (2) to the port device (1), and
- a second connector (11) arranged at a second end (141) of the channel (14) for connecting a second medical line (3) to the port device (1),
**characterized by**
- a port (12) for connecting an injection device (4) to the port device (13) for injecting a medical fluid into the channel (14), the port (12) having a conduit (124) being in fluid connection with the channel (14), and
- a closing element (15) which in a first state separates the conduit (124) of the port (12) from the channel (14) and is elastically deformable from the first state to allow a fluid passage from the conduit (124) into the channel (14).

2. Port device (1) according to claim 1, **characterized in that** the closing element (15) comprises a closing pin (151) which, in the first state, reaches into the conduit (124) of the port (12).

3. Port device (1) according to claim 2, **characterized in that** the closing pin (151), in a second state of the closing element (15), is removed from the conduit (124) to allow a fluid passage from the conduit (124) into the channel (14).

4. Port device (1) according to one of claims 1 to 3, **characterized in that** the closing element (15) comprises a body section (150), the body section (150) being elastically connected to the body (13) of the port device (1).

5. Port device (1) according to claim 4, **characterized in that** the closing element (15) comprises a membrane section (152) elastically connecting the body section (150) to the body (13) of the port device (1).

6. Port device (1) according to claim 5, **characterized in that** the membrane section (152) comprises at least one opening (153) for allowing a fluid passage from the conduit (124) into the channel (14).

7. Port device (1) according to claim 5 or 6, **characterized in that** the membrane section (152) is connected, along a circumferential fastening edge (154), inside the channel (14) to the body (13) of the port device (1).

8. Port device (1) according to one of the preceding claims, **characterized in that** the closing element (15) is arranged at an end of the conduit (124) leading into the channel (14).

9. Port device (1) according to one of the preceding claims, **characterized in that** the port (12) comprises a pin (123) in which the conduit (124) is formed, the pin (123) being engageable with a connector (40) of the injection device (4).

10. Port device (1) according to one of the preceding claims, **characterized in that** the conduit (124) extends along a substantially transverse direction with respect to the channel (14).

11. Port device (1) according to one of the preceding claims, **characterized in that** the closing element (15) is deformable by an injection pressure caused when injecting a medical fluid through the conduit (124), or mechanically by a pin (401) of the injection device (4) inserted into the conduit (124).
